(19) Europäisches Patentamt
European Patent Office
Office européen des brevets

(11) Publication number : **0 514 207 A2**

(12) **EUROPEAN PATENT APPLICATION**

(21) Application number : **92304432.5**

(22) Date of filing : **15.05.92**

(51) Int. Cl.$^5$ : **C12N 15/19,** C12N 15/85, C07K 13/00, G01N 33/68, C12Q 1/02

(30) Priority : **17.05.91 US 701930**
**17.05.91 US 701937**
**17.05.91 US 701935**

(43) Date of publication of application : **19.11.92 Bulletin 92/47**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Strader, Catherine D.**
**119 Morningside Road**
**Verona, NJ 07044 (US)**
Inventor : **Fong, Tung Ming**
**38 Bolton Court**
**Somerset, NJ 08873 (US)**

(74) Representative : **Thompson, John Dr. et al**
**Merck & Co., Inc. European Patent Department Terlings Park Eastwick Road**
**Harlow, Essex CM20 2QR (GB)**

(54) **Novel human neurokinin-1 receptor short form.**

(57) A novel recombinant human neurokinin-1 receptor short form (hereinafter identified as human NK1R sF) is disclosed which has been prepared by polymerase chain reaction techniques. Also disclosed is the complete sequence of human NK1R sF complementary DNA ; expression systems, including a CHO (chinese hamster ovarian cell line) stable expression system ; and an assay using the CHO expression system.

NK1R sF, can be used in an assay to identify and evaluate entities that bind substance P receptor or NK1R sF. The assay cap also be used in conjunction with diagnosis and therapy to determine the body fluid concentration of substance P antagonists in arthritis patients.

FIGURE 3

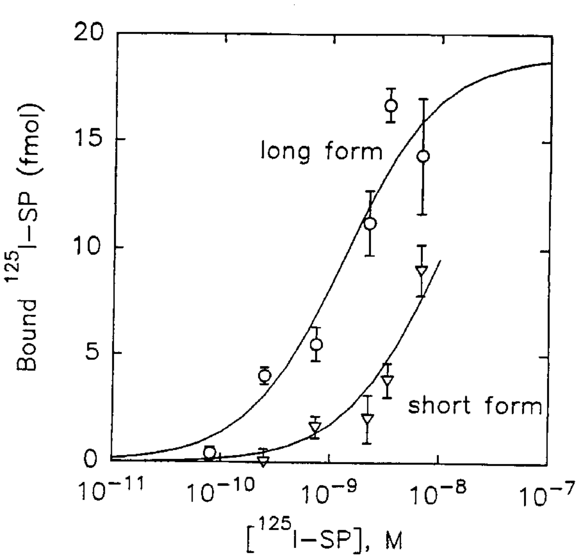


EP 0 514 207 A2

## BACKGROUND OF THE INVENTION

The present invention concerns a novel cloned human neurokinin-1 receptor short form (hereinafter identified as human NK1R sF) and recombinant human NK1R sF.

J. Yokota, et al., J. Biol. Chem., 264:17649 (1989) have reported cloned rat neurokinin-1 receptor. N.P. Gerard, et al., J. Biol. Chem., 265:20455 (1990), have reported human neurokinin-2 receptor. Cloned rat and bovine neurokinin-2 receptor have likewise been reported. See respectively, Y. Sasi, and S. Nakanishi, Biochem Biophys. Res. Comm., 165:695 (1989), and Y. Masu, et al., Nature 329:836 (1987). Cloned rat neurokinin-3 receptor has also been reported by R. Shigemoto, et al., J. Biol. Chem., 265:623 (1990).

The above references, however, neither disclose or suggest the instant invention.

Substance P is a naturally occuring undecapeptide belonging to the tachykinin family of peptides. Substance P is a pharmacologically-active neuropeptide that is produced in mammals. Its characteristic amino acid sequence is illustrated in U.S. 4,680,283. As is well known in the art substance P and other tachykinins have been implicated in the pathophysiology of numerous diseases. Substance P has been shown to be involved in the transmission of pain or migraine (see B.E.B. Sandberg et al., Journal of Medicinal Chemistry, Vol. 25, p. 1009 (1982)), as well as in central nervous system disorders such as anxiety and schizophrenia, in respiratory and inflammatory diseases such as asthma and rheumatoid arthritis, respectively, and in gastrointestinal disorders and diseases of the GI tract, like ulcerative colitis and Crohn's disease, etc. (see D. Regoli in "Trends in Cluster Headache," edited by F. Sicuteri et al., Elsevier Scientific Publishers, Amsterdam, 1987, pp. 85-95).

The instant invention also concerns an assay protocol which can be used to determine the activity in body fluids of substances that bind human NK1R or NK1R sF; these include substance P . The assay can also be used for identifying and evaluating substances that bind NK1R or NK1R sF. Thus, the assay can be used to identify substance P antagonists and evaluate their binding affinity. Other methods includes that described by M.A. Cascieri, et al., J. Biol. Chem., 258-5158 (1983). By use of such methods, substance P antagonists have been identified. See, for example, R. M. Snider, et al., Science, 251:435 (Jan. 1991) and S. McLean, et al., Science, 251:437 (Jan. 1991). See also W090/05525 which published May 31, 1990, which is hereby incorporated by reference. Methods to date have proven inferior, in part, for failure of the animal receptor (animal NK1R, NK2R or NK3R) activity to accurately reflect that of human neurokinin-1 receptor. Furthermore, prior to this disclosure human NK1R has not been available in a purified form or in substantial isolation from NK2R and/or NK3R. Use of such neurokinin receptor sources can not accurately depict the affinity for a human NK1R, and in particular, NK1R sF.

## BRIEF DESCRIPTION OF THE DRAWINGS

Figure 1 Full length amino acid sequence of human neurokinin-1 receptor short form.

Figure 2 Full length nucleotide sequence of the cloned human neurokinin-1 receptor short form complementary DNA.

Figure 3 Binding affinity of neurokinin-1 receptor long (NK1R) and short forms (NK1RSF) for substance P (SP) in COS assay.

Figure 4 Activating phospholipase C as mediated by neurokinin-1 receptor short and long forms.

## SUMMARY OF THE INVENTION

A novel recombinant human neurokinin-1 receptor short form (hereinafter identified as human NK1R sF) is disclosed which has been prepared by polymerase chain reaction techniques. Also disclosed is the complete sequence of human NK1R sF complementary DNA; expression systems, including a CHO (chinese hamster ovarian cell line) stable expression system; and an assay using the CHO expression system.

NK1R sF, can be used in an assay to identify and evaluate entities that bind substance P receptor or NK1R sF. The assay can also be used in conjunction with diagnosis and therapy to determine the body fluid concentration of substance P antagonists in arthritis patients.

## DETAILLED DESCRIPTION OF THE INVENTION

One embodiment of the invention concerns human neurokinin-1 receptor short form, said receptor being free of other human receptor proteins.

In one class this embodiment concerns human neurokinin-1 receptor short form, said receptor being free of other human proteins.

Within this class, this embodiment concerns human neurokinin-1 receptor short form from human cells such as glioblastoma, said receptor being free of other human proteins.

In a second class, this embodiment concerns a protein comprising the 311 amino acid sequence depicted in Figure 1, said protein being free of other human receptor proteins.

Within the second class this embodiment concerns a protein consisting of the 311 amino acid sequence as shown in Figure 1.

The first embodiment also concerns a pharmaceutical composition for inhibiting the binding of substance P to cellular neurokinin-1 receptor or a cellular neurokinin-1 receptor short form, said composition comprising an effective amount of neurokinin-1 receptor short form.

The first embodiment also concerns a method of inhibiting the binding of substance P to cellular human neurokinin-1 receptor or a cellular neurokinin-1 receptor short form, in a patient in need of such inhibition, comprising: administration of an effective amount of human neurokinin-1 receptor short form.

The use of such pharmaceutical compositions and methods for antagonising the binding of substance P to in vivo neurokinin-1 receptor is disclosed in, for example, R. M. Snider, et al., Science, 251:435 (Jan. 1991); S. McLean, et al., Science, 251:437 (Jan. 1991); and PCT Patent application W090/05525 which published May 31, 1990. The administration of an effective amount of human neurokinin-1 receptor short form may be conducted utilizing essentially the same procedure.

A second embodiment concerns a DNA sequence encoding human neurokinin receptor short form complementary DNA, said DNA, said sequence being free of other human DNA sequences.

As will be appreciated by those of skill in the art, there is a substantial amount of redundancy in the set of codons which translate specific amino acids. Accordingly, the invention also includes alternative base sequences wherein a codon (or codons) are replaced with another codon, such that the amino acid sequence translated by the DNA sequence remains unchanged. For purposes of this specification, a sequence bearing one or more such replaced codons will be defined as a degenetate variation. Also included are mutations (exchange of individual amino acids) which one of skill in the art would expect to have no effect on functionality, such as valine for leucine, arginine for lysine and asparagine for glutamine.

One class of the second embodiment of the invention concerns the nucleotide sequence of complementary DNA, beginning with nucleotide 123 and ending with necleotide 1055 as shown in Figure 2.

Within this class of the second embodiment is the DNA sequence (SEQ ID NO:1:) that further comprises:

```
        10         20         30         40         50         60         70
GAAAAAGCCT TCCACCCTCC TGTCTGGCTT TAGAAGGACC CTGAGCCCCA GGCGCCACGA CAGGACTCTG

        80         90        100        110        120 122
CTGCAGAGGG GGGTTGTGTA CAGATAGTAG GGCTTTACCG CCTAGCTTCG AA
```

or a degenerate variation thereof.

The second embodiment the invention concerns the partial nucleotide sequence of complementary DNA, as shown in Figure 2 or a degenerate variation thereof.

A third embodiment of this invention concerns systems for expressing human neurokinin-1 receptor short form.

One class this third embodiment of the invention comprises:

A plasmid which comprises:

(a) a mammalian expression vector, such as pRcCMV, and

(b) a base sequence encoding human neurokinin-1 receptor short form protein.

Within this class of the third embodiment the neurokinin-1 receptor short form comprises the nucleotide sequence of complementary DNA, beginning with nucleotide 123 and ending with necleotide 1055 as shown in Figure 2.

A second class of this third embodiment of the invention concerns a system for the transient expression of human neurokinin-1 receptor short form in a monkey kidney cell line (COS), the system comprising a vector comprising human neurokinin receptor short form (human NK1R sF) cDNA.

A third class of this third embodiment of the invention concerns a system for the expression of human neurokinin-1 receptor short form in a chinese hamster ovarian cell line (CHO), the system comprising a vector comprising human neurokinin receptor short form (human NK1R sF) cDNA.

Within this class of the third embodiment is is the sub-class wherein the expression system includes

A plasmid which comprises:

(a) a mammalian expression vector, such as pRcCMV, and

(b) a base sequence encoding human neurokinin-1 receptor short form protein.

Within this sub-class the neurokinin-1 receptor expression system comprises the nucleotide sequence of complementary DNA, beginning with nucleotide 123 and ending with necleotide 1055 as shown in Figure 2. It is understood, and is readily apparent to those skilled in the art that a wide variety of commonly used mammalian cell lines are suitable for use in the present invention. Suitable cell lines derived from mammalian species include, but are not limited to, cell lines of human, bovine, porcine, monkey, and rodent origin.

A forth embodiment of the invention concerns a method of using any of the above expression systems for determining the binding affinity of a test sample for human neurokinin-1 receptor and/or NK1R sF.

In one class this embodiment concerns a method of using a Chinese hamster ovarian cell line (CHO), said line transplanted with a plasmid, which plasmid comprises:

(a) a mammalian expression vector, such as pRcCMV, and

(b) the base sequence encoding human neurokinin-1 receptor short form protein, the method which comprises:

(1) expressing human neurokinin-1 receptor short form in said CHO cells;

(2) addition of a test sample to a solution containing $^{125}$I-substance P and said cells;

(3) incubating the products of Step (2), said incubation effective for expressing said the human neurokinin-1 receptor short form and effective for competitive binding of said $^{125}$I-substance P and said test sample to said human neurokinin-1 receptor short form;

(4) separating said $^{125}$I-substance P which is bound to said human neurokinin-1 receptor short form from said $^{125}$I-substance P which is not bound;

(5) measuring the radioactivity of said $^{125}$I-substance P which is bound to said human neurokinin-1 receptor short form.

In a second class this embodiment concerns a method of using a monkey kidney cell line (COS), said line transplanted with.a plasmid, which plasmid comprises:

(a) a mammalian expression vector, such as pCMD8, and

(b) the base sequence encoding human neurokinin-1 receptor short form protein, the method which comprises:

(1) expressing human neurokinin-1 receptor short form in said COS cells;

(2) addition of a test sample to a solution containing $^{125}$I-substance P and said cells;

(3) incubating the products of Step (2), said incubation effective for expressing said the human neurokinin-1 receptor short form and effective for competitive binding of said $^{125}$I-substance P and said test sample to said human neurokinin-1 receptor short form;

(4) separating said $^{125}$I-substance P which is bound to said human neurokinin-1 receptor short form from said $^{125}$I-substance P which is not bound;

(5) measuring the radioactivity of said $^{125}$I-substance P which is bound to said human neurokinin-1 receptor short form.

In a third class this embodiment concerns a method of using a Chinese hamster ovarian cell line (CHO), said line transplanted with a plasmid which plasmid comprises

(a) a mammalian expression vector, such as pRcCMV, and

(b) the base sequence encoding human neurokinin-1 receptor short form protein, the method comprising:

(1) expressing human neurokinin-I receptor short form in said CHO cells;

(2) equilibrating the product of Step (1) with $^{3}$H-myoinositol;

(3) washing the product of Step (2);

(4) incubating the product of Step (3) with a test sample in the presence of 10 mM LiCl, which results in the production of inositol monophosphate;

(5) measuring the inositol monophosphate.

In overview, the present invention describes methods to isolate the human neurokinin-1 receptor short form (human NK1R sF) complementary DNA (cDNA) without prior knowledge of its protein sequence or gene sequence. Human NK1R and NFIR sF are membrane receptors for the neurotransmitter substance P. Polymerase chain reaction (PCR) technique was utilized for the isolation of human NK1R sF cDNA. In the approach, the regions of rat NK1R applicants thought to be similar to human NK1R were identified, oligonucleotide primers corresponding to those region were designed, PCR amplification was carried out to obtain part of the NK1R sF cDNA from human cells, and its DNA sequence was determined. The remaining part of the human NK1R sF cDNA was obtained from a human cDNA library utilizing the above sequence information of human NK1R sF cDNA.

The complete sequence of the human NK1R sF cDNA was determined, and its encoded protein sequence was deduced. Among other things, such sequence information is useful in the process of developing novel substance P antagonists.

Three heterologous expression systems were used to express the cloned human NK1R sF cDNA. The Xenopus oocyte expression enahles one to determine the biological function of human NK1R sF . The COS (a monkey kidney cell line) expression can be used to measure the ligand binding properties of human NK1R sF. The CHO (a Chinese hamster ovarian cell line) stable expression is suitable for natural product screen to identify potential therapeutic agent or other substances that bind to substance P receptor or human NK1R sF This cell line can also be used as an assay kit for determining the body fluid concentration of substance P in arthritis patients.

Assay protocols use the heterologously expressed human NK1R sF for determination of the binding affinity and antagonistic activity of substance P antagonists.

1) Isolation of human NK1R sF cDNA

As will be appreciated by those of skill in the art, NK1R has been believed to exist as a single type. Accordingly, a strategy was devised to isolalate that single NK1R type. Surpisingly, the applicants unexpectedly found both a full length NK1R and a NK1R short form the latter which is believed to constitute a previously unknown receptor sub-type.

To isolate the human NK1R cDNA in the absence of its sequence information, we developed methods to obtain three separate but overlapping cDNA clones in three steps. (i) We have adopted the homologous cloning strategy (Ohara et al., 1989, Proc. Nat. Acad. Sci., 86:5673-5677) to isolate cDNA clones encoding the central core region of human NK1R, with the assumption that the human NK1R sequence is similar to the published sequence (Yokota et al., 1989, J. Biol. Chem., 264:17649-17652) of rat NK1R in certain areas where appropriate PCR primels can be designed. Degenerate primers corresponding to the rat sequence were used in PCR amplification (Mullis and Faloona, 1987, Meth. Enzymol., 155:335) to obtain the cDNA encoding the central trnsmembrane core region of human NK1R from human mRNA. (ii) After determining the sequence of the core region in human NK1R, new primers corresponding to the human sequence were designed and a second homologous PCR amplification was performed using the human primer in the core region with degenerate primers corresponding to the N-terminal sequence of rat NK1R. The cDNA encoding the N-terminal region of human NK1R was thus obtained from human mRNA and its sequence was determined. (iii) An anchored PCR strategy was developed to isolate the cDNA encoding the C-terminal region of human NK1R, in which primers corresponding to the core region of human NK1R were used in combination with a primer corresponding to the sequence of a cloning vector to obtain the cDNA from a human cDNA library.

By use of this strategy, both NK1R and NK1R sF were isolated. To confirm the authenticity of the cDNA encoding human NK1R sF, an independent PCR amplification was performed to obtain the full length cDNA in a single step using primers from the 5′ and 3′ untranslated regions.

2) Expression of the cloned human NK1R sF

Three expression systems were developed for the cloned human NK1R sF. An transient expression in Xenopus oocytes resulted from microinjection of in vitro transcribed mRNA from the cloned cDNA (Xenopus Laevis from XENOPUS ONE, Ann Arbor, MI). This system allows the measurement of biological effect of NK1R sF activation upon ligand-binding. Another transient expression in COS (a monkey kidney cell line, ATCC CRL 1651, ATCC Rockville MD) resulted from the transfection of the cloned cDNA under the control of viral promoter into mammalian cells (e.g., COS). The transfected cells are suitable for determination binding affinity of human NK1R sF for various ligands. Stable expression of human NK1R sF in mammalian cells (e.g., CHO, a Chinese hamster ovarian cell line, ATCC CRL 9096, ATCC Rockville MD) was achieved after integration of the transfected cDNA into the chromosomes of the host cells. These stable cell lines will constituently express the cloned human NK1R sF and can be propagated infinitely. Therefore, stable expression system is very useful in large scale drug screen, and can be used to determine substance p concentration in the biopsy sample of patients.

To establish a stable cell line expressing the cloned human NK1R sF, the cDNA was subcloned into the vector pRcCMV (INVITROGEN).

The electrophysiological assay of human NK1R sF expressed in Xenopus oocytes was based on the fact that NK1R activates the phospholipase C upon substance P binding, and phospholipase C in turn increases the intracellular calcium concentration through inositol trisphosphate ($IP_3$) and $IP_3$-gated calcium channel on intracellular membranes. The calcium increase activates calcium-gated chloride channels on plasma membranes which gives rise to a chloride current measurable by two electrode voltage clamp.

The binding assay of human NK1R sF expressed in COS or CHO is based on the use of $^{125}$I-substance P ($^{125}$I-SP, from DU PONT, Boston, MA) as a radioactively labeled ligand which compete with unlabeled substance P or any other ligand for binding to the human NK1R sF. Monolayer cell culture of COS or CHO was dissociated by the non-enzymatic solution (SPECIALTY MEDIA, Lavallette, NJ) and resuspended in appropriate volume of the binding buffer (50 mM Tris pH 7.5, 5 mM $MnCl_2$, 150 mM NaCl, 0.04 mg/ml bacitracin, 0.004 mg/ml leupeptin, 0.2 mg/ml BSA, 0.01 mM phosphoramidon).

The activation of phospholipase C by NK1R sF can also be measured in CHO cells by determining the accumulation of inositol monophosphate which is a degradation product of $IP_3$.

In addition to large scale drug screening using the stable CHO cell line expressing the cloned human NK1R sF, other alternative applications will become apparent to those of skill in the art obvious. For example, the stable cell line can be used in the binding assay to determine the substance P concentration from biopsy samples. The human NK1R sF protein can also be injected into patients to reduce substance P concentration in some neurogenic inflammatory diseases.

The following examples are given for the purpose of illustrating the present invention and shall not be construed as being limitations on the scope or spirit of the present invention.

EXAMPLE 1

Step A:

In the first step of obtaining the cDNA encoding the central core region of human NK1R sF, human mRNA was prepared from three human glioblastoma cell lines T98G, CCF-STTG1 and U87MG (obtained from the American Type Culture Collection, Rockville, MD) by the FASTTRACK method (INVITROGEN, San Diego, CA). Synthesis of first strand cDNA from 4 ug of human mRNA was initiated by oligo (dT) primers in a total volume of 20 ul according to protocols of the BRL cDNA synthesis system (BRL, LIFE TECHNOLOGIES, Inc., Gaithersburg, MD). Ten ul of the first strand cDNA was used as template with three rat primers (50 pmol rspr2s4, 50 pmol rspr2s4h, and 100 pmol rspr7a2; see Table I for their sequences) in a primary PCR amplification in a total volume of 100 ul according to the GENEAMP protocol (PERKIN ELMER CETUS, Norwalk, CT). Thirty cycles of PCR were performed using the following parameters: 1 min of denaturation at 94°C, 2 min of annealing at 40°C and 4 min of extension at 72°C with 2 sec of auto extension. Ten ul of the primary PCR product was used as template with the same primers in a secondary PCR amplification under the same cycling conditions to further amplify the DNA. Ten ul of the secondary PCR product was used as template with three rat primers (50 pmol rspr2s4, 50 pmol rspr2s4h, 50 pmol rspr7al and 50 pmol rspr7alh) in 30 cycles of tertiary PCR amplification with the following parameters: 1 min of denaturation at 94°C, 2 min of annealing at 45°C, and 4 min of extension at 72°C with 2 sec of auto extension. The tertiary PCR product was analyzed by agarose gel electrophoresis and was found to contain a 600 bp DNA fragment. This DNA fragment was excised from the gel, purified by GENECLEAN (Bio 101, La Jolla, CA), phosphorylated, and subcloned into Sma I site of the plasmid vector BLUESCRIPT SK+ (STRATAGENE, La Jolla, CA). The DNA sequence was determined by the Sequenase dideoxy chain termination method (USBC, Cleveland, OH). Sequence alignment analysis showed that this cDNA fragment is similar (90% identity at nucleotide level) to the central core region of rat NK1R from amino acid 91 to 280.

STEP B:

After determination of the core region sequence of human NK1R sF, five antisense primers were synthesized based on the human sequence (hspr3a5, hspr5al, hspr5a2, hspr6al and hspr6a2; see Table II for their sequences). These primers would be used to obtain the N-terminal cDNA sequence of human NK1R sF. One ug of human glioblastoma MRNA and 6 uM of each of the above primers was used in first strand cDNA synthesis in a total volume of 20 ul according the BRL cDNA synthesis protocols. The cDNA was extracted by phenol-chloroform, precipitated by ethanol and dissolved in 30 ul of water. Ten ul of the cDNA was used as template with two rat primers (50 pmol rsprn and 50 pmol rsprnh) and one human primer (150 pmol hspr3a5) in the primary PCR amplification in a total volume of 100 ul. Thirty cycles were performed with the following parameters: 1 min denaturation at 94°C, 1 min of annealing at 55°C, and 3 min of extension at 72°C. Five ul of the primary PCR product was then used as template with two rat primers (50 pmol rsprn and 50 pmol rsprnh) and one human primer (100 pmol hspr3a4) in 30 cycles of secondary PCR amplification with the same parameters. Two ul of the secondary PCR product was used as template with two rat primers (50 pmol rsprn and 50 pmol rsprnh) and one human primer in 30 cycles of tertiary PCR amplification with the same parameters. The tertiary PCR product was analyzed by agarose gel electrophoresis and was found to contain a 500 bp fragment. This DNA fragment can hybridize with a human oligonucleotide (hspr3a2), indicating it is not a non-specific by-product. This DNA fragment was excised from the gel, purified by GENECLEAN (Bio 101), phosphorylated, and subcloned into Sma I site of the vector Bluescript SK+. DNA sequence analysis revealed that this fragment encodes the human NK1R sF N-terminal region and it also contains 51 untranslated sequence.

STEP C:

In the third step, an anchored PCR protocol was developed in which the cDNA encoding the C-terminal region of human NK1R sF was obtained from a cDNA library using sense human primers and a primer corresponding to the vector sequence. Three ug of human glioblastoma mRNA was primed by 2.5 ug of oligo (dT) in the first strand cDNA synthesis in a total volume of 50 ul, followed by second strand cDNA synthesis according the BRL cDNA synthesis protocols. The cDNA product was then heated at 70°C for 10 min. The yield of double stranded cDNA was determined by incorporating 1.25 uM of $^{32}$p-a-dCTP as tracer in the reaction. Four ul of T4 DNA polymerase was added to the reaction mixture and incubated at 37°C for 10 min. The reaction was stopped by adding 16 ul of 250 mm EDTA, extracting with phenol/$CHCl_3$, and precipitating with ethanol. The cDNA was dissolved in 50 ul of HE buffer (10 mm HEPES-1mM EDTA). Small size cDNA was removed by the Select-D(RF) SPIN COLUMN (5′TO3′, Boulder, CO), and the large size cDNA was Precipitated by ethanol and dissolved in 36 ul of water. Four ul of 0.2 M Tris-10 mM spermidine-1 mM EDTA (pH7.5) was added to the tube and heated at 70°C for 1 min. The cDNA was phosphorylated by adding 5 ul of blunt-end kinase buffer (0.5 M Tris pH 9.5, 0.1 M $MgCl_2$, 50 mM DTT, 50% glycerol), 2.5 ul of 10 mM ATP, 2.5 ul of polynucleotide kinase, and incubating at 37°C for 30 min. The cDNA was extracted by phenol/$CHCl_3$, precipitated by ethanol and ligated to EcoRI linker according to the PROMEGA ECORI linker ligation protocol (PROMEGA, Madison, WI). Linker-ligated cDNA was then ligated to calf intestinal phosphatase-treated EcoRI site of the vector BLUESCRIPT SK+. One ul of the ligated plasmid DNA was used as template in 30 cycles of primary PCR with two human primers (50 pmol hspr6sl and 50 pmol hspr6s2) and 100 pmol of vector-specific primer t3 (obtained from STRATAGENE) with the following parameters: 1 min of denaturation at 94°C, 2 min of annealing at 55°C, and 4 min of extension at 72°C with 2 sec auto extension. One ul of the primary PCR product was used in 30 cycles of secondary PCR amplification with one human primer (100 pmol hspr6s3) and the same vector-specific primer t3 under the same conditions. One ul of the secondary PCR product was used in 30 cycles of tertiary PCR amplification with one human primer (100 pmol hspr6s4) and 100 pmol of vector-specific primer SK (STRATAGENE) under the same conditions. A 400 bp DNA fragment was detected which also hybridized to a human oligo probe hspr6s5. This DNA fragment was excised from the agarose gel, purified by GENECLEAN (BIO 101), phosphorylated, and subcloned into Sma I site of the vector BLUESCRIPT SK+. DNA sequence analysis revealed that it encodes the C-terminal region of human NK1R sF amd contains 3′ untranslated sequence.

STEP D:

Since three separate but overlapping cDNA clones encoding human NK1R sF were isolated above and the possibility of alternative pre-mRNA splicing exists, it is necessary to confirm the authenticity of the full length cDNA sequence by isolating a full length cDNA directly. Based on the above sequence in the untranslated region, primers were synthesized which should give rise to a full length cDNA. Using the PERKIN ELMER CETUS RNA PCR amplification kit (Perkin Elmer Cetus), cDNA was synthesized from 1.5 ug of human glioblastoma mRNA in a total volume of 20 ul with 50 pmol of the human primer hsprl2utal. One half of the first strand cDNA was used as template in 30 cycles of primary PCR amplification with two human primers (50 pmol hsprl2utal, 50 pmol hspr5utsl) with the following parameters: 1 min of denaturation at 94°C, 2 min of annealing at 55°C, and 4 min of extension at 55°C with 2 sec auto extension. Ten ul of the primary PCR product was used as template in 30 cycles of secondary PCR amplification with two human primers (50 pmol hsprl2uta2 and 50 pmol hspr5uts2) under the same conditions. A 1100 bp DNA fragment was excised from agarose gel, purified by GENECLEAN (BIO 101), digested with restriction endonucleases with EcoRI and Not I, and subcloned into the vector BLUESCRIPT SK+. DNA sequence analysis confirmed the general structure of the cloned human NK1R cDNA sF. The sequence of human NK1R sF cDNA is shown in Figure 2.

Table I: Primers based on rat NK1R sequence. The last letter "h" in some primers denotes that human codon bias was incorporated (Lathe, 1985, J. Mol. Biol., 183:1-12). The position number in the rat cDNA sequence was defined by Yokota et al. (J. Biol. Chem., 1989, 264:17649-17652).

| Name | Sequence (SEQ ID NO:_:) | Position | Direction |
|---|---|---|---|
| rspr2s4 | TGCATGGCTGCATTCAAT (2) | 238 - 255 | sense |
| rspr2s4h | TGCATGGCTGCCTTCAA (3) | 238 - 254 | sense |
| rspr7a2 | ACAGTAGATGATGGGGTTGTACAT (4) | 918 - 894 | antisense |
| rspr7a1 | CAGGTAGACCTGCTGGATGAACTT (5) | 864 - 841 | antisense |
| rspr7a1h | CAGGTACACCTGCTGGATGAACTT (6) | 864 - 841 | antisense |
| rsprn | ATGGATAACGTCCTTCCTAT (7) | 1 - 20 | sense |
| rsprnh | ATGGACAATGTGCTGCCCA (8) | 1 - 19 | sense |

Table II: Primers based on the human NK1R cDNA sF sequence. Position number is defined in the sequence listing in the text. The nucleotides in parentheses are not present in the human NK1R sF cDNA; they are restriction sites for subcloning purpose.

| Name | Sequence (SEQ ID NO:_:) | Position | Direction |
|---|---|---|---|
| hspr3a2 | GAAGAAGTTGTGGAACTTGCA (9) | 455 - 435 | antisense |
| hspr3a1 | CATGGAGTAGATACTGGCGAA (10) | 491 - 471 | antisense |
| hspr3a4 | GGATGTATGATGGCCATGTA (11) | 532 - 513 | antisense |
| hspr3a5 | ACTTTGGTGGCTGTGGCTGA (12) | 568 - 549 | antisense |
| hspr5a1 | ATGCATAGCCAATCACCAGCA (13) | 768 - 748 | antisense |
| hspr5a2 | CATAGTGTGATTCCCACTAC (14) | 793 - 774 | antisense |
| hspr6a1 | TGCACACCACGACAATCATCA (15) | 888 - 868 | antisense |
| hspr6a2 | TTGATGTAGGGCAGGAGGAA (16) | 943 - 924 | antisense |
| hspr6s1 | GCAAGTCTCTGCCAAGCGCAA (17) | 836 - 856 | sense |
| hspr6s2 | TGATGATTGTCGTGGTGTGCA (18) | 868 - 888 | sense |
| hspr6s3 | TTCCACATCTTCTTCCTCCT (19) | 912 - 931 | sense |
| hspr6s4 | CTACATCAACCCAGATCTCT (20) | 935 - 954 | sense |
| hspr6s5 | TCTCTACCTGAAGAAGTT (21) | 950 - 967 | sense |
| hspr12uta1 | TCACAGCTGGGTTTACTCAT (22) | 1050 - 1131 | antisense |
| hspr12uta2 | (GACATGCGGCCGC)CGCTTGGCCACTGTGTAT (23) | 1121 - 1104 | antisense |
| hspr5uts1 | CCTCCTGTCTGGCTTTAGAA (24) | 16 - 35 | sense |
| hspr5uts2 | (GCGCAGAATTC)GTGTACAGATAGTAGGCTT (25) | 86 - 105 | sense |

EXAMPLE 2

Expression in Xenopus oocytes

To express the human NK1R sF cDNA in Xenopus oocytes, the cDNA was cloned into an in vitro transcription vector BLUESCRIPT SK+ (STRATAGENE) which contains the T7 promoter for initiation of T7 RNA polymerase catalyzed RNA synthesis. One ug of linear plasmid DNA which contained the human NK1R cDNA downstream of the T7 promoter was used in the in vitro transcription reaction containing 40 mm Tris pH 7.5, 50 mM NaCl, 8 mM $MgCl_2$, 2 mM spermidine, 0.4 mm CTP, 0.4 mM ATP, 0.4 mM UTP, 0.16 mM GTP, 2.5 ul CAP analog (STRATAGENE), 30 mM DTT, 1 U RNase Block II (STRATAGENE), 0.83 pmol 32 P-A-CTP and 25 U of T7 RNA polymerase. The reaction tube was incubated at 37°C for 1 hour. Usually 5 ug of RNA was synthesized as quantitated by incorporation of $^{32}$p-A-CTP into RNA. After RNA synthesis, the plasmid DNA was removed by adding 10 U of RNase free DNase and 1 U of RNase Block II. The reaction mixture was extracted by phenol/$CHCl_3$, and the unincorporated nucleotides were removed by the Select-D(RF) spin column (5′TO3′). The RNA transcript was precipitated by ethanol twice and dissolved in RNase free water. Oocytes were removed from Xenopus frogs, treated with 2 mg/ml collagenase (specific activity < 0.3 U/mg, BOEHRINGER MANNHEIM, Indianapolis, IN) in OR-2 buffer (82.5 mM NaCl, 2 mm KCl, 1 mm $MgCl_2$, 5 mM HEPES, pH 7.4) for 4 hours at 19°C. The dissociated oocytes were incubated in OR-2 buffer supplemented by 1.8 mM $CaCl_2$, 0.5 mg/ml gentamycin and 0.5 mM theophylline at 19°C overnight before injection. A 50 nl aliquot contain 2 ng of RNA transcript was injected into each oocyte. The injected oocytes were incubated at 19°C for 2 days before electrophysiological recording (see Example 3 for assay method).

Expression in COS

To express the human NK1R sF transiently in COS, the cDNA was cloned into the expression vector pCDM9 which was derived from pCDM8 (INVITROGEN) by inserting the ampicillin resistance gene (nucleotide 1973 to 2964 from BLUESCRIPT SK+) into the Sac II site. Transfection of 20 ug of the plasmid DNA into 10 millions COS cells was achieved by electroporation in 800 ul of transfection buffer (135 mm NaCl, 1.2 MM $CaCl_2$, 1.2 mM $MgCl_2$) 2.4 mM $K_2HPO_4$, 0.6 mM $KH_2PO_4$, 10 mM glucose, 10 mM HEPES pH 7.4) at 260 V and 950 uF using the IBI GENEZAPPER (IBI, New Haven, CT). The cells were incubated in 10% fetal calf serum, 2 mM glutamine, 100U/ml penicillin-streptomycin, and 90% DMEM media (GIBCO, Grand Island, NY) in 5% $CO_2$ at 37°C for three days before the binding assay.

Stable Expression in CHO

To establish a stable cell line expressing the cloned human NK1R sF, the cDNA is subcloned into the vector pRcCMV (INVITROGEN). Transfection of 20 ug of the plasmid DNA into CHO cells is achieved by electroporation in 800 ul of transfection buffer suplemented with 0.625 mg/ml Herring sperm DNA at 300 V and 950 uF using the IBI GENEZAPPER (IBI). The transfected cells are incubated in CHO media [10 % fetal calf serum, 100 U/ml pennicilin-streptomycin, 2 mM glutamine, 1/500 hypoxanthine-thymidine (ATCC), 90% IMDM media (JRH BIOSCIENCES, Lenexa, KS), 0.7 mg/ml G418 (GIBCO)] in 5% $CO_2$ at 37°C until colonies are visible. Each colony was separated and propagated. The cell clone with the highest number of human NK1R is selected for subsequent application in the assay of Example 3.

EXAMPLE 3

Assay Protocol Using Oocytes

The oocyte was voltage-clamped at - 80 mV by the model 8500 intracellular preamp-clamp (DAGAN, Minneapolis, MN). The recoding chamber was continuously perfused with recording buffer (96 mM NaCl, 2 mM KCl, 1.8 mM $CaCl_2$, 5 mM HEPES, pH 7.4). Chloride current was elicited by applying substance P (from 0.1 nM to 1000 nM) to the recording chamber. At least three oocytes were measured for each concentration. The antagonistic activity of any potential substance P antagonist can be assessed by determining the inhibition of substance P response. Likewise, NK1R and NK1R sF agonists can be identified by their ability to stimulate a response in oocytes injected with NK1R sF mRNA but not in uninjected oocytes. As shown in Figure 4, the activation of phospholipase C mediated by the short and long forms of NK1R also exhibit a differential sensitivity to the agonist substance P.

Assay Protocol using COS or CHO

The binding assay of human NK1R sF expressed in COS (or CHO) is based on the use of $^{125}$I-substance P ($^{125}$I-SP, from DU PONT, Boston, MA) as a radioactively labeled ligand. Monolayer cell culture of COS (or CHO) was dissociated by the non-enzymatic solution (SPECIALTY MEDIA, Lavallette, NJ) and resuspended in appropriate volume of the binding buffer (50 mm Tris pH 7.5, 5 mM $MnCl_2$, 150 mM NaCl, 0.04 mg/ml bacitracin, 0.004 mg/ml leupeptin, 0.2 mg/ml BSA, 0.01 mM phosphoramidon) such that 200 ul of the cell suspension would give rise to about 1000 cpm of specific $^{125}$I-SP binding at 6.7 nM. In the binding assay, 200 ul of cells were added to a tube containing 20 ul binding buffer and 200 ul of various serial dilution of $^{125}$I-SP stock solution. The $^{125}$I-SP stock solution was at 14 nM with a specific activity of 40 cpm/fmol. The non-specific binding was determined by including 20 ul of 210 uM unlabeled SP instead of buffer in the above assay mixture. The mixture was incubated at 4°C for 1 hour with gentle shaking. The bound radioactivity was separated from unbound radioactivity by GF/C filter (BRANDEL, Gaithersburg, MD) which was pre-wetted with 0.1 % polyethylenimine. The filter was washed with 3 ml of wash buffer (50 mM Tris pH 7.5, 5 mM $MnCl_2$, 150 mM NaCl) three times and its radioactivity was determined by gamma counter. Illustrative of this method of using these expression systems are the results shown in Figure 3. These results show the difference in binding affinity of substance P for the short and long forms of human NK1R.

ALTERNATIVE PROTOCOL

The activation of phospholipase C by NK1R sF may also be measured in CHO cells by determining the accumulation of inositol monophosphate which is a degradation product of $IP_3$. CHO cells are seeded in 12-well plate at 250,000 cells per well. After incubating in CHO media for 4 days, cells are loaded with 0.025 uCi/ml of $^3$H-myoinositol by overnight incubation. The extracellular radioactivity is removed by washing with phosphate buffered saline. LiCl is added to the well at final concentration of 0.1 mm with or without antagonist, and continued incubation at 37°C for 15 min. Substance P is added to the well at final concentration of 0.3 nM to activate the human NK1R sF. After 30 min of incubation at 37°C, the media is removed and 0.1 N HCl is added. Each well is sonicated at 4°C and extracted with $CHCl_3$/methanol (1:1). The aqueous phase is applied to a 1 ml Dowex AG 1X8 ion exchange column. The column is washed with 0.1 N formic acid followed by 0.025 M ammonium formate-0.1 N formic acid. The inositol monophosphate is eluted with 0.2 M ammonium formate-0.1 N formic acid and quantitated by beta counter.

While the foregoing specification teaches the principles of the present invention, with examples provided for the purpose of illustration, it will be understood that the practice of the invention encompasses all of the casual variations, adaptioations, modifications, deletions, or additions of procedures and protocols described herein, as come within the scope of the following claims and its equivalents.

SEQUENCE LISTING

(2) INFORMATION FOR SEQ ID NO:1:

(i) SEQUENCE CHARACTERISTICS:
(A) LENGTH: 122 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:1:

GAAAAAGCCT TCCACCCTCC TGTCTGGCTT TAGAAGGACC CTGAGCCCCA GGCGCCACGA 60

CAGGACTCTG CTGCAGAGGG GGGTTGTGTA CAGATAGTAG GGCTTTACCG CCTAGCTTCG 120

AA 122

(2) INFORMATION FOR SEQ ID NO:2:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:2:

TGCATGGCTG CATTCAAT 18

(2) INFORMATION FOR SEQ ID NO:3:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:3:

TGCATGGCTG CCTTCAA 17

(2) INFORMATION FOR SEQ ID NO:4:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:4:

ACAGTAGATG ATGGGGTTGT ACAT 24

(2) INFORMATION FOR SEQ ID NO:5:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:5:

CAGGTAGACC TGCTGGATGA ACTT 24

(2) INFORMATION FOR SEQ ID NO:6:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:6:

CAGGTACACC TGCTGGATGA ACTT 24

(2) INFORMATION FOR SEQ ID NO:7:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:7:

ATGGATAACG TCCTTCCTAT 20

(2) INFORMATION FOR SEQ ID NO:8:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:8:

ATGGACAATG TGCTGCCCA 19

(2) INFORMATION FOR SEQ ID NO:9:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:9:

GAAGAAGTTG TGGAACTTGC A 21

(2) INFORMATION FOR SEQ ID NO:10:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:10:

CATGGAGTAG ATACTGGCGA A 21

(2) INFORMATION FOR SEQ ID NO:11:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:11:

GGATGTATGA TGGCCATGTA 20

(2) INFORMATION FOR SEQ ID NO:12:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:12:

ACTTTGGTGG CTGTGGCTGA 20

(2) INFORMATION FOR SEQ ID NO:13:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:13:

ATGCATAGCC AATCACCAGC A 21

(2) INFORMATION FOR SEQ ID NO:14:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:14:

CATAGTGTGA TTCCCACTAC 20

(2) INFORMATION FOR SEQ ID NO:15:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:15:

TGCACACCAC GACAATCATC A 21

(2) INFORMATION FOR SEQ ID NO:16:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:16:

TTGATGTAGG GCAGGAGGAA 20

(2) INFORMATION FOR SEQ ID NO:17:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:17:

GCAAGTCTCT GCCAAGCGCA A 21

(2) INFORMATION FOR SEQ ID NO:18:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:18:

TGATGATTGT CGTGGTGTGC A 21

(2) INFORMATION FOR SEQ ID NO:19:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:19:

TTCCACATCT TCTTCCTCCT 20

(2) INFORMATION FOR SEQ ID NO:20:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:20:

CTACATCAAC CCAGATCTCT 20

(2) INFORMATION FOR SEQ ID NO:21:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:21:

TCTCTACCTG AAGAAGTT 18

(2) INFORMATION FOR SEQ ID NO:22:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:22:

TCACAGCTGG GTTTACTCAT 21

(2) INFORMATION FOR SEQ ID NO:23:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:23:

CGCTTGGCCA CTGTGTAT 18

(2) INFORMATION FOR SEQ ID NO:24:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:24:

CCTCCTGTCT GGCTTTAGAA 20

(2) INFORMATION FOR SEQ ID NO:25:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE:

(xi) SEQUENCE DESCRIPTION: SEQ ID NO:25:

GTGTACAGAT AGTAGGCTT 19

(2) INFORMATION FOR SEQ ID NO:26:

(i) SEQUENCE CHARACTERISTICS:
(A) LENGTH: 311 amino acids
(B) TYPE: amino acid
(C) STRANDEDNESS: single
(D) TOPOLOGY: linear

(ii) MOLECULE TYPE:

(xi) SEQUENCE DESCRIPTION: SEQ ID NO:26:

```
Met Asp Asn Val Leu Pro Val Asp Ser Asp Leu Ser Pro Asn Ile Ser
1               5                   10                  15

Thr Asn Thr Ser Glu Pro Asn Gln Phe Val Gln Pro Ala Trp Gln Ile
            20                  25                  30

Val Leu Trp Ala Ala Ala Tyr Thr Val Ile Val Val Thr Ser Val Val
        35                  40                  45

Gly Asn Val Val Val Met Trp Ile Ile Leu Ala His Lys Arg Met Arg
    50                  55                  60

Thr Val Thr Asn Tyr Phe Leu Val Asn Leu Ala Phe Ala Glu Ala Ser
65                  70                  75                  80

Met Ala Ala Phe Asn Thr Val Val Asn Phe Thr Tyr Ala Val His Asn
                85                  90                  95
```

```
Glu Trp Tyr Tyr Gly Leu Phe Tyr Cys Lys Phe His Asn Phe Phe Pro
            100                 105                 110

Ile Ala Ala Val Phe Ala Ser Ile Tyr Ser Met Thr Ala Val Ala Phe
        115                 120                 125

Asp Arg Tyr Met Ala Ile Ile His Pro Leu Gln Pro Arg Leu Ser Ala
    130                 135                 140

Thr Ala Thr Lys Val Val Ile Cys Val Ile Trp Val Leu Ala Leu Leu
145                 150                 155                 160

Leu Ala Phe Pro Gln Gly Tyr Tyr Ser Thr Thr Glu Thr Met Pro Ser
                165                 170                 175

Arg Val Val Cys Met Ile Glu Trp Pro Glu His Pro Asn Lys Ile Tyr
            180                 185                 190

Glu Lys Val Tyr His Ile Cys Val Thr Val Leu Ile Tyr Phe Leu Pro
        195                 200                 205

Leu Leu Val Ile Gly Tyr Ala Tyr Thr Val Val Gly Ile Thr Leu Trp
    210                 215                 220

Ala Ser Glu Ile Pro Gly Asp Ser Ser Asp Arg Tyr His Glu Gln Val
225                 230                 235                 240

Ser Ala Lys Arg Lys Val Val Lys Met Met Ile Val Val Val Cys Thr
                245                 250                 255

Phe Ala Ile Cys Trp Leu Pro Phe His Ile Phe Phe Leu Leu Pro Tyr
            260                 265                 270

Ile Asn Pro Asp Leu Tyr Leu Lys Lys Phe Ile Gln Gln Val Tyr Leu
        275                 280                 285

Ala Ile Met Trp Leu Ala Met Ser Ser Thr Met Tyr Asn Pro Ile Ile
    290                 295                 300

Tyr Cys Cys Leu Asn Asp Arg
305                 310
```

(2) INFORMATION FOR SEQ ID NO:27:

(i) SEQUENCE CHARACTERISTICS:
- (A) LENGTH: 1269 base pairs
- (B) TYPE: nucleic acid
- (C) STRANDEDNESS: single
- (D) TOPOLOGY: linear

(ii) MOLECULE TYPE:

(xi) SEQUENCE DESCRIPTION: SEQ ID NO:27:

```
GAAAAAGCCT TCCACCCTCC TGTCTGGCTT TAGAAGGACC CTGAGCCCCA GGCGCCACGA     60
CAGGACTCTG CTGCAGAGGG GGGTTGTGTA CAGATAGTAG GGCTTTACCG CCTAGCTTCG    120
AAATGGATAA CGTCCTCCCG GTGGACTCAG ACCTCTCCCC AAACATCTCC ACTAACACCT    180
CGGAACCCAA TCAGTTCGTG CAACCAGCCT GGCAAATTGT CCTTTGGGCA GCTGCCTACA    240
CGGTCATTGT GGTGACCTCT GTGGTGGGCA ACGTGGTAGT GATGTGGATC ATCTTAGCCC    300
ACAAAAGAAT GAGGACAGTG ACGAACTATT TTCTGGTGAA CCTGGCCTTC GCGGAGGCCT    360
CCATGGCTGC ATTCAATACA GTGGTGAACT TCACCTATGC TGTCCACAAC GAATGGTACT    420
ACGGCCTGTT CTACTGCAAG TTCCACAACT TCTTCCCCAT CGCCGCTGTC TTCGCCAGTA    480
TCTACTCCAT GACGGCTGTG GCCTTTGATA GGTACATGGC CATCATACAT CCCCTCCAGC    540
CCCGGCTGTC AGCCACAGCC ACCAAAGTGG TCATCTGTGT CATCTGGGTC CTGGCTCTCC    600
TGCTGGCCTT CCCCCAGGGC TACTACTCAA CCACAGAGAC CATGCCCAGC AGAGTCGTGT    660
GCATGATCGA ATGGCCAGAG CATCCGAACA AGATTTATGA GAAAGTGTAC CACATCTGTG    720
TGACTGTGCT GATCTACTTC CTCCCCCTGC TGGTGATTGG CTATGCATAC ACCGTAGTGG    780
GAATCACACT ATGGGCCAGT GAGATCCCCG GGGACTCCTC TGACCGCTAC CACGAGCAAG    840
TCTCTGCCAA GCGCAAGGTG GTCAAAATGA TGATTGTCGT GGTGTGCACC TTCGCCATCT    900
GCTGGCTGCC CTTCCACATC TTCTTCCTCC TGCCCTACAT CAACCCAGAT CTCTACCTGA    960
AGAAGTTTAT CCAGCAGGTC TACCTGGCCA TCATGTGGCT GGCCATGAGC TCCACCATGT   1020
ACAACCCCAT CATCTACTGC TGCCTCAATG ACAGGTGAGG ATCCCAACCC CATGAGCTCT   1080
CCAGGGGCCA CAAGACCATC TACATACACA GTGGCCAAGC GGCATCCTAA ATGAGTAAAC   1140
CCAGCTGTGA GACAAGAGGG ACAAGTGGGG ACTGCAGCTA ACTTATCATC ACACAACTCA   1200
GCCTGGCTGA TTATCACCAT CCAGGAATGG GAGCCCGGAG TAGACTGATT TTCTTTTTTT   1260
CTTTTCCAC                                                          1269
```

## Claims

1. A human neurokinin-1 receptor short form, said receptor being substantially free of other human receptor proteins.

2. A protein corresponding to the amino acid sequence of human neurokinin-1 receptor short form, said protein consisting of 311 amino acids.

3. A protein corresponding to the amino acid sequence (SEQ ID NO:26:) which is:

```
                                             10                                                    20
Met Asp Asn Val Leu Pro Val Asp Ser Asp Leu Ser Pro Asn Ile Ser Thr Asn Thr Ser

                                             30                                                    40
Glu Pro Asn Gln Phe Val Gln Pro Ala Trp Gln Ile Val Leu Trp Ala Ala Ala Tyr Thr

                                             50                                                    60
Val Ile Val Val Thr Ser Val Val Gly Asn Val Val Val Met Trp Ile Ile Leu Ala His

                                             70                                                    80
Lys Arg Met Arg Thr Val Thr Asn Tyr Phe Leu Val Asn Leu Ala Phe Ala Glu Ala Ser

                                             90                                                   100
Met Ala Ala Phe Asn Thr Val Val Asn Phe Thr Tyr Ala Val His Asn Glu Trp Tyr Tyr

                                            110                                                   120
Gly Leu Phe Tyr Cys Lys Phe His Asn Phe Phe Pro Ile Ala Ala Val Phe Ala Ser Ile

                                            130                                                   140
Tyr Ser Met Thr Ala Val Ala Phe Asp Arg Tyr Met Ala Ile Ile His Pro Leu Gln Pro

                                            150                                                   160
Arg Leu Ser Ala Thr Ala Thr Lys Val Val Ile Cys Val Ile Trp Val Leu Ala Leu Leu

                                            170                                                   180
Leu Ala Phe Pro Gln Gly Tyr Tyr Ser Thr Thr Glu Thr Met Pro Ser Arg Val Val Cys

                                            190                                                   200
Met Ile Glu Trp Pro Glu His Pro Asn Lys Ile Tyr Glu Lys Val Tyr His Ile Cys Val

                                            210                                                   220
Thr Val Leu Ile Tyr Phe Leu Pro Leu Leu Val Ile Gly Tyr Ala Tyr Thr Val Val Gly

                                            230                                                   240
Ile Thr Leu Trp Ala Ser Glu Ile Pro Gly Asp Ser Ser Asp Arg Tyr His Glu Gln Val

                                            250                                                   260
Ser Ala Lys Arg Lys Val Val Lys Met Met Ile Val Val Val Cys Thr Phe Ala Ile Cys

                                            270                                                   280
Trp Leu Pro Phe His Ile Phe Phe Leu Leu Pro Tyr Ile Asn Pro Asp Leu Tyr Leu Lys

                                            290                                                   300
Lys Phe Ile Gln Gln Val Tyr Leu Ala Ile Met Trp Leu Ala Met Ser Ser Thr Met Tyr

                                            310
Asn Pro Ile Ile Tyr Cys Cys Leu Asn Asp Arg      .
```

4. A DNA sequence, encoding human neurokinin-1 receptor short form, said sequence being free of other human DNA sequences.

5. A DNA sequence comprising the sequence (SEQ ID NO:28:) which is:

```
                                                                     130        140
                                                               ATGGATAA CGTCCTCCCG

       150        160        170        180        190        200        210
GTGGACTCAG ACCTCTCCCC AAACATCTCC ACTAACACCT CGGAACCCAA TCAGTTCGTG CAACCAGCCT

       220        230        240        250        260        270        280
GGCAAATTGT CCTTTGGGCA GCTGCCTACA CGGTCATTGT GGTGACCTCT GTGGTGGGCA ACGTGGTAGT

       290        300        310        320        330        340        350
GATGTGGATC ATCTTAGCCC ACAAAAGAAT GAGGACAGTG ACGAACTATT TTCTGGTGAA CCTGGCCTTC

       360        370        380        390        400        410        420
GCGGAGGCCT CCATGGCTGC ATTCAATACA GTGGTGAACT TCACCTATGC TGTCCACAAC GAATGGTACT

       430        440        450        460        470        480        490
ACGGCCTGTT CTACTGCAAG TTCCACAACT TCTTCCCCAT CGCCGCTGTC TTCGCCAGTA TCTACTCCAT

       500        510        520        530        540        550        560
GACGGCTGTG GCCTTTGATA GGTACATGGC CATCATACAT CCCCTCCAGC CCCGGCTGTC AGCCACAGCC

       570        580        590        600        610        620        630
ACCAAAGTGG TCATCTGTGT CATCTGGGTC CTGGCTCTCC TGCTGGCCTT CCCCCAGGGC TACTACTCAA

       640        650        660        670        680        690        700
CCACAGAGAC CATGCCCAGC AGAGTCGTGT GCATGATCGA ATGGCCAGAG CATCCGAACA AGATTTATGA

       710        720        730        740        750        760        770
GAAAGTGTAC CACATCTGTG TGACTGTGCT GATCTACTTC CTCCCCCTGC TGGTGATTGG CTATGCATAC

       780        790        800        810        820        830        840
ACCGTAGTGG GAATCACACT ATGGGCCAGT GAGATCCCCG GGGACTCCTC TGACCGCTAC CACGAGCAAG

       850        860        870        880        890        900        910
TCTCTGCCAA GCGCAAGGTG GTCAAAATGA TGATTGTCGT GGTGTGCACC TTCGCCATCT GCTGGCTGCC

       920        930        940        950        960        970        980
CTTCCACATC TTCTTCCTCC TGCCCTACAT CAACCCAGAT CTCTACCTGA AGAAGTTTAT CCAGCAGGTC

       990       1000       1010       1020       1030       1040       1050
TACCTGGCCA TCATGTGGCT GGCCATGAGC TCCACCATGT ACAACCCCAT CATCTACTGC TGCCTCAATG

 1055
ACAGG
```

or a degenerate variation thereof.

6. A DNA sequence comprising the sequence (SEQ ID NO:27:) which is:

```
        10         20         30         40         50         60         70
GAAAAAGCCT TCCACCCTCC TGTCTGGCTT TAGAAGGACC CTGAGCCCCA GGCGCCACGA CAGGACTCTG

        80         90        100        110        120        130        140
CTGCAGAGGG GGGTTGTGTA CAGATAGTAG GGCTTTACCG CCTAGCTTCG AAATGGATAA CGTCCTCCCG

       150        160        170        180        190        200        210
GTGGACTCAG ACCTCTCCCC AAACATCTCC ACTAACACCT CGGAACCCAA TCAGTTCGTG CAACCAGCCT

       220        230        240        250        260        270        280
GGCAAATTGT CCTTTGGGCA GCTGCCTACA CGGTCATTGT GGTGACCTCT GTGGTGGGCA ACGTGGTAGT

       290        300        310        320        330        340        350
GATGTGGATC ATCTTAGCCC ACAAAAGAAT GAGGACAGTG ACGAACTATT TTCTGGTGAA CCTGGCCTTC

       360        370        380        390        400        410        420
GCGGAGGCCT CCATGGCTGC ATTCAATACA GTGGTGAACT TCACCTATGC TGTCCACAAC GAATGGTACT

       430        440        450        460        470        480        490
ACGGCCTGTT CTACTGCAAG TTCCACAACT TCTTCCCCAT CGCCGCTGTC TTCGCCAGTA TCTACTCCAT

       500        510        520        530        540        550        560
GACGGCTGTG GCCTTTGATA GGTACATGGC CATCATACAT CCCCTCCAGC CCCGGCTGTC AGCCACAGCC

       570        580        590        600        610        620        630
ACCAAAGTGG TCATCTGTGT CATCTGGGTC CTGGCTCTCC TGCTGGCCTT CCCCCAGGGC TACTACTCAA

       640        650        660        670        680        690        700
CCACAGAGAC CATGCCCAGC AGAGTCGTGT GCATGATCGA ATGGCCAGAG CATCCGAACA AGATTTATGA

       710        720        730        740        750        760        770
GAAAGTGTAC CACATCTGTG TGACTGTGCT GATCTACTTC CTCCCCCTGC TGGTGATTGG CTATGCATAC

       780        790        800        810        820        830        840
ACCGTAGTGG GAATCACACT ATGGGCCAGT GAGATCCCCG GGGACTCCTC TGACCGCTAC CACGAGCAAG

       850        860        870        880        890        900        910
TCTCTGCCAA GCGCAAGGTG GTCAAAATGA TGATTGTCGT GGTGTGCACC TTCGCCATCT GCTGGCTGCC

       920        930        940        950        960        970        980
CTTCCACATC TTCTTCCTCC TGCCCTACAT CAACCCAGAT CTCTACCTGA AGAAGTTTAT CCAGCAGGTC

       990       1000       1010       1020       1030       1040       1050
TACCTGGCCA TCATGTGGCT GGCCATGAGC TCCACCATGT ACAACCCCAT CATCTACTGC TGCCTCAATG

      1060       1070       1080       1090       1100       1110       1120
ACAGGTGAGG ATCCCAACCC CATGAGCTCT CCAGGGGCCA CAAGACCATC TACATACACA GTGGCCAAGC

      1130       1140       1150       1160       1170       1180       1190
GGCATCCTAA ATGAGTAAAC CCAGCTGTGA GACAAGAGGG ACAAGTGGGG ACTGCAGCTA ACTTATCATC

      1200       1210       1220       1230       1240       1250       1260
ACACAACTCA GCCTGGCTGA TTATCACCAT CCAGGAATGG GAGCCCGGAG TAGACTGATT TTCTTTTTTT

CTTTTCCAC
```

or a degenerate variation thereof.

7. A plasmid which comprises:
   (a) a mammalian cell vector, and
   (b) a base sequence encoding human neurokinin-1 receptor short form protein

8. A plasmid which comprises:
   (a) vector pRcCMV, and
   (b) the base sequence (SEQ ID NO:28:) which is:

```
                                                                        130        140
                                                                   ATGGATAA CGTCCTCCCG

       150        160        170        180        190        200        210
GTGGACTCAG ACCTCTCCCC AAACATCTCC ACTAACACCT CGGAACCCAA TCAGTTCGTG CAACCAGCCT

       220        230        240        250        260        270        280
GGCAAATTGT CCTTTGGGCA GCTGCCTACA CGGTCATTGT GGTGACCTCT GTGGTGGGCA ACGTGGTAGT

       290        300        310        320        330        340        350
GATGTGGATC ATCTTAGCCC ACAAAAGAAT GAGGACAGTG ACGAACTATT TTCTGGTGAA CCTGGCCTTC

       360        370        380        390        400        410        420
GCGGAGGCCT CCATGGCTGC ATTCAATACA GTGGTGAACT TCACCTATGC TGTCCACAAC GAATGGTACT

       430        440        450        460        470        480        490
ACGGCCTGTT CTACTGCAAG TTCCACAACT TCTTCCCCAT CGCCGCTGTC TTCGCCAGTA TCTACTCCAT

       500        510        520        530        540        550        560
GACGGCTGTG GCCTTTGATA GGTACATGGC CATCATACAT CCCCTCCAGC CCCGGCTGTC AGCCACAGCC

       570        580        590        600        610        620        630
ACCAAAGTGG TCATCTGTGT CATCTGGGTC CTGGCTCTCC TGCTGGCCTT CCCCCAGGGC TACTACTCAA

       640        650        660        670        680        690        700
CCACAGAGAC CATGCCCAGC AGAGTCGTGT GCATGATCGA ATGGCCAGAG CATCCGAACA AGATTTATGA

       710        720        730        740        750        760        770
GAAAGTGTAC CACATCTGTG TGACTGTGCT GATCTACTTC CTCCCCCTGC TGGTGATTGG CTATGCATAC

       780        790        800        810        820        830        840
ACCGTAGTGG GAATCACACT ATGGGCCAGT GAGATCCCCG GGGACTCCTC TGACCGCTAC CACGAGCAAG

       850        860        870        880        890        900        910
TCTCTGCCAA GCGCAAGGTG GTCAAAATGA TGATTGTCGT GGTGTGCACC TTCGCCATCT GCTGGCTGCC

       920        930        940        950        960        970        980
CTTCCACATC TTCTTCCTCC TGCCCTACAT CAACCCAGAT CTCTACCTGA AGAAGTTTAT CCAGCAGGTC

       990       1000       1010       1020       1030       1040       1050
TACCTGGCCA TCATGTGGCT GGCCATGAGC TCCACCATGT ACAACCCCAT CATCTACTGC TGCCTCAATG

 1055
ACAGG
```

or a degenerate variation thereof.

9. A method of using a mammalian cell line, said line transfected with a plasmid which plasmid comprises:
   (a) a mammalian expression vector, and

(b) the base sequence encoding human neurokinin-1 receptor short form protein,
the method comprising:
(1) expressing human neurokinin-1 receptor short form in said mammalian cells;
(2) addition of a test sample to a solution containing $^{125}$I-substance P and said plasmid;
(3) incubating the products of Step 2, wherein said incubation allows the binding of said $^{125}$I-substance P and said test sample to said human neurokinin receptor short form;
(4) separation of said $^{125}$I-substance P which is bound to said human neurokinin-1 receptor short form from said $^{125}$I-substance P which is not bound;
(5) measuring the amount of said $^{125}$I-substance P which is bound to said human neurokinin-1 receptor short form.

**10.** A method of using a mammalian cell line, said line transplanted with a plasmid which plasmid comprises:
(a) a mammalian expression vector, and
(b) the base sequence encoding human neurokinin-1 receptor short form protein,
the method comprising:
(1) expressing human neurokinin-I receptor in said mammalian cells;
(2) equilibrating the product of Step (1) with [$^3$H]-myoinositol;
(3) washing the product of Step (2);
(4) incubating the product of Step (3) with a test sample in the presence of aqueous LiCl, which results in the production of [$^3$H]-inositol monophosphate;
(5) measuring the [$^3$H]-inositol monophosphate

FIGURE 1

(SEQ ID NO: 26:)

10 20
Met Asp Asn Val Leu Pro Val Asp Ser Asp Leu Ser Pro Asn Ile Ser Thr Asn Thr Ser

30 40
Glu Pro Asn Gln Phe Val Gln Pro Ala Trp Gln Ile Val Leu Trp Ala Ala Ala Tyr Thr

50 60
Val Ile Val Val Thr Ser Val Val Gly Asn Val Val Val Met Trp Ile Ile Leu Ala His

70 80
Lys Arg Met Arg Thr Val Thr Asn Tyr Phe Leu Val Asn Leu Ala Phe Ala Glu Ala Ser

90 100
Met Ala Ala Phe Asn Thr Val Val Asn Phe Thr Tyr Ala Val His Asn Glu Trp Tyr Tyr

110 120
Gly Leu Phe Tyr Cys Lys Phe His Asn Phe Phe Pro Ile Ala Ala Val Phe Ala Ser Ile

130 140
Tyr Ser Met Thr Ala Val Ala Phe Asp Arg Tyr Met Ala Ile Ile His Pro Leu Gln Pro

150 160
Arg Leu Ser Ala Thr Ala Thr Lys Val Val Ile Cys Val Ile Trp Val Leu Ala Leu Leu

170 180
Leu Ala Phe Pro Gln Gly Tyr Tyr Ser Thr Thr Glu Thr Met Pro Ser Arg Val Val Cys

190 200
Met Ile Glu Trp Pro Glu His Pro Asn Lys Ile Tyr Glu Lys Val Tyr His Ile Cys Val

210 220
Thr Val Leu Ile Tyr Phe Leu Pro Leu Leu Val Ile Gly Tyr Ala Tyr Thr Val Val Gly

230 240
Ile Thr Leu Trp Ala Ser Glu Ile Pro Gly Asp Ser Ser Asp Arg Tyr His Glu Gln Val

250 260
Ser Ala Lys Arg Lys Val Val Lys Met Met Ile Val Val Val Cys Thr Phe Ala Ile Cys

270 280
Trp Leu Pro Phe His Ile Phe Phe Leu Leu Pro Tyr Ile Asn Pro Asp Leu Tyr Leu Lys

290 300
Lys Phe Ile Gln Gln Val Tyr Leu Ala Ile Met Trp Leu Ala Met Ser Ser Thr Met Tyr

310
Asn Pro Ile Ile Tyr Cys Cys Leu Asn Asp Arg

FIGURE 2

(SEQ ID NO:27:)

10 20 30 40 50 60 70
GAAAAAGCCT TCCACCCTCC TGTCTGGCTT TAGAAGGACC CTGAGCCCCA GGCGCCACGA CAGGACTCTG

80 90 100 110 120 130 140
CTGCAGAGGG GGGTTGTGTA CAGATAGTAG GGCTTTACCG CCTAGCTTCG AAATGGATAA CGTCCTCCCG

150 160 170 180 190 200 210
GTGGACTCAG ACCTCTCCCC AAACATCTCC ACTAACACCT CGGAACCCAA TCAGTTCGTG CAACCAGCCT

220 230 240 250 260 270 280
GGCAAATTGT CCTTTGGGCA GCTGCCTACA CGGTCATTGT GGTGACCTCT GTGGTGGGCA ACGTGGTAGT

290 300 310 320 330 340 350
GATGTGGATC ATCTTAGCCC ACAAAAGAAT GAGGACAGTG ACGAACTATT TTCTGGTGAA CCTGGCCTTC

360 370 380 390 400 410 420
GCGGAGGCCT CCATGGCTGC ATTCAATACA GTGGTGAACT TCACCTATGC TGTCCACAAC GAATGGTACT

430 440 450 460 470 480 490
ACGGCCTGTT CTACTGCAAG TTCCACAACT TCTTCCCCAT CGCCGCTGTC TTCGCCAGTA TCTACTCCAT

500 510 520 530 540 550 560
GACGGCTGTG GCCTTTGATA GGTACATGGC CATCATACAT CCCCTCCAGC CCCGGCTGTC AGCCACAGCC

570 580 590 600 610 620 630
ACCAAAGTGG TCATCTGTGT CATCTGGGTC CTGGCTCTCC TGCTGGCCTT CCCCCAGGGC TACTACTCAA

640 650 660 670 680 690 700
CCACAGAGAC CATGCCCAGC AGAGTCGTGT GCATGATCGA ATGGCCAGAG CATCCGAACA AGATTTATGA

710 720 730 740 750 760 770
GAAAGTGTAC CACATCTGTG TGACTGTGCT GATCTACTTC CTCCCCCTGC TGGTGATTGG CTATGCATAC

780 790 800 810 820 830 840
ACCGTAGTGG GAATCACACT ATGGGCCAGT GAGATCCCCG GGGACTCCTC TGACCGCTAC CACGAGCAAG

850 860 870 880 890 900 910
TCTCTGCCAA GCGCAAGGTG GTCAAAATGA TGATTGTCGT GGTGTGCACC TTCGCCATCT GCTGGCTGCC

920 930 940 950 960 970 980
CTTCCACATC TTCTTCCTCC TGCCCTACAT CAACCCAGAT CTCTACCTGA AGAAGTTTAT CCAGCAGGTC

990 1000 1010 1020 1030 1040 1050
TACCTGGCCA TCATGTGGCT GGCCATGAGC TCCACCATGT ACAACCCCAT CATCTACTGC TGCCTCAATG

1060 1070 1080 1090 1100 1110 1120
ACAGGTGAGG ATCCCAACCC CATGAGCTCT CCAGGGGCCA CAAGACCATC TACATACACA GTGGCCAAGC

1130 1140 1150 1160 1170 1180 1190
GGCATCCTAA ATGAGTAAAC CCAGCTGTGA GACAAGAGGG ACAAGTGGGG ACTGCAGCTA ACTTATCATC

1200 1210 1220 1230 1240 1250 1260
ACACAACTCA GCCTGGCTGA TTATCACCAT CCAGGAATGG GAGCCCGGAG TAGACTGATT TTCTTTTTTT

CTTTTCCAC

FIGURE 3

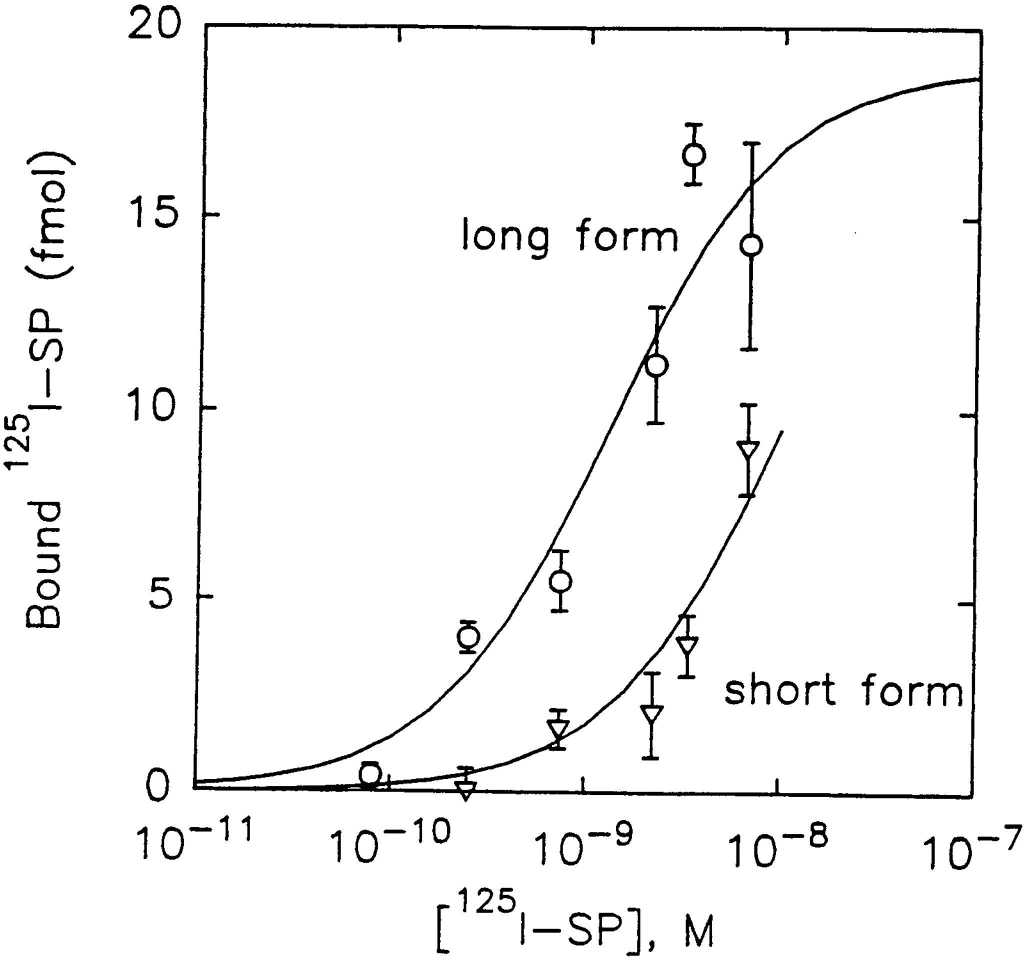
20
15
10
5
0
Bound $^{125}$I−SP (fmol)
long form
short form
$10^{-11}$
$10^{-10}$
$10^{-9}$
$10^{-8}$
$10^{-7}$
[$^{125}$I−SP], M

FIGURE 4

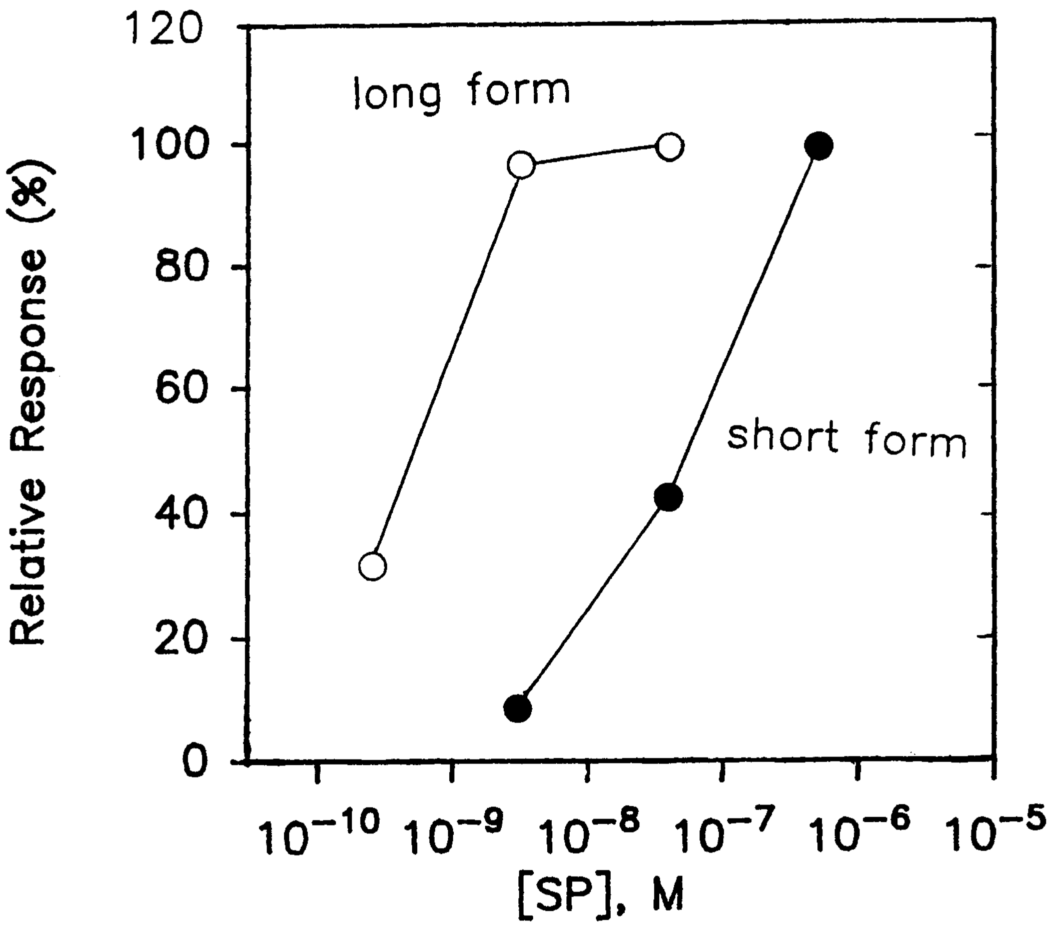
120
100
80
60
40
20
0
long form
short form
Relative Response (%)
$10^{-10}$ $10^{-9}$ $10^{-8}$ $10^{-7}$ $10^{-6}$ $10^{-5}$
[SP], M